(19) Europäisches Patentamt
European Patent Office
Office européen des brevets

(11) Veröffentlichungsnummer: **0 268 923**
**A1**

(12) **EUROPÄISCHE PATENTANMELDUNG**

(21) Anmeldenummer: **87116564.3**

(22) Anmeldetag: **10.11.87**

(51) Int. Cl.⁴: **C07D 275/06**, C07D 317/28, C07D 307/14, C07D 327/04, C07D 405/06, C07D 411/06, C07D 413/06, A01N 43/80, A01N 43/40, A01N 43/28, A01N 43/84

(30) Priorität: **22.11.86 DE 3639903**

(43) Veröffentlichungstag der Anmeldung:
**01.06.88 Patentblatt 88/22**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Krämer, Wolfgang, Dr.**
**Rosenkranz 25**
**D-5093 Burscheid 2(DE)**
Erfinder: **Reinecke, Paul, Dr.**
**Steinstrasse 8**
**D-5090 Leverkusen 3(DE)**
Erfinder: **Hänssler, Gerd, Dr.**
**Am Arenzberg 58a**
**D-5090 Leverkusen 3(DE)**

(54) **Saccharin-Salze von Amino-methylheterocyclen.**

(57) Saccharin-Salze von Aminomethylheterocyclen der allgemeinen Formel (I),

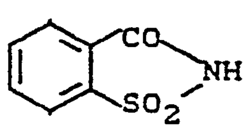

x

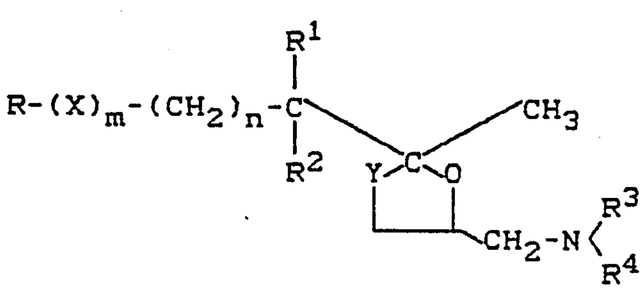

(I)

in welcher

R bis $R^4$, m, n, X und Y die in der Beschreibung gegebene Bedeutung haben und ihre Verwendung in Schädlingsbekämpfungsmitteln.

Die neuen Saccharin-Salze der Aminomethylheterocyclen der Formel (I) können aus geeigneten Aminomethylheterocyclen und Saccharin hergestellt werden, gegebenenfalls in Gegenwart eines Lösungsmittels.

EP 0 268 923 A1

## Saccharin-Salze von Aminomethylheterocyclen

Die Erfindung betrifft neue Saccharin-Salze von Aminomethylheterocyclen, ein Verfahren zu ihrer Herstellung und ihre Verwendung als Schädlingsbekämpfungsmittel.

Es ist bereits bekannt, daß Saccharin-Salze von Aminoheterocyclen, wie beispielsweise das Saccharin-Salz des 5-Amino-1,2,4-triazols fungizide Eigenschaften besitzen (vgl. EP 158 074).

Außerdem ist bekannt, daß bestimmte Aminomethylheterocyclen, wie beispielsweise das 2-[1-(2-Methylphenoxy)-2-methyl-prop-2-yl]-2-methyl-4-(piperidin-1-yl-methyl)-1,3-dioxolan ebenfalls fungizide Eigenschaften besitzen (vgl. z. B. EP 97 822).

Deren Wirkung ist jedoch insbesondere bei niedrigen Aufwandmengen und Konzentrationen nicht in allen Anwendungsgebieten völlig zufriedenstellend.

Es wurden neue Saccharin-Salze von Aminomethylheterocyclen der allgemeinen Formel (I),

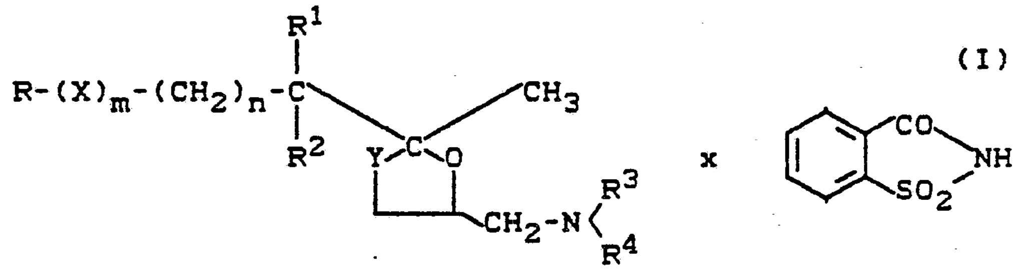

(I)

in welcher

R für gegebenenfalls substituiertes Cycloalkyl, für gegebenenfalls substituiertes Aryl oder für gegebenenfalls substituiertes Thienyl steht,

$R^1$ für Wasserstoff oder Alkyl steht,

$R^2$ für Alkyl steht,

$R^3$ für Alkyl steht und

$R^4$ für Alkyl, Alkenyl oder Alkinyl steht, oder

$R^3$ und $R^4$ gemeinsam mit dem Stickstoffatom, an welches sie gebunden sind, für einen gegebenenfalls substituierten, gesättigten Heterocyclus stehen, der weitere Heteroatom enthalten kann,

X für Sauerstoff oder Schwefel steht,

Y für Sauerstoff, Schwefel oder die Methylengruppe steht,

m für 0 oder 1 steht und

n für 0 oder 1 steht,

gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Saccharin-Salze von Aminomethylheterocyclen der allgemeinen Formel (I),

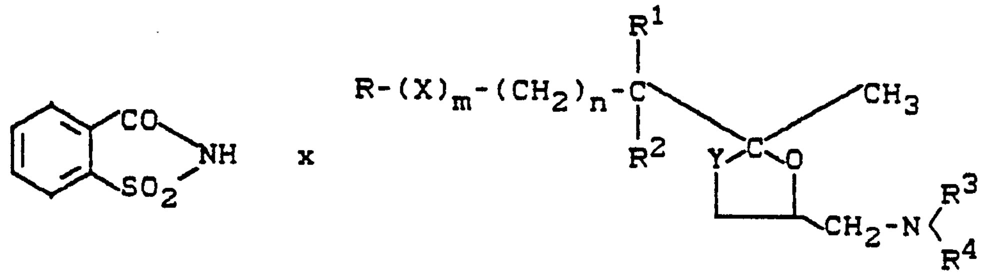

x

(I)

in welcher

R für gegebenenfalls substituiertes Cycloalkyl, für gegebenenfalls substituiertes Aryl oder für gegebenenfalls substituiertes Thienyl steht,

$R^1$ für Wasserstoff oder Alkyl steht,

$R^2$ für Alkyl steht,

$R^3$ für Alkyl steht und

$R^4$ für Alkyl, Alkenyl oder Alkinyl steht, oder

$R^3$ und $R^4$ gemeinsam mit dem Stickstoffatom, an welches sie gebunden sind, für einen gegebenenfalls substituierten, gesättigten Heterocyclus stehen, der weitere Heteroatome enthalten kann,

X für Sauerstoff oder Schwefel steht,

Y für Sauerstoff, Schwefel oder die Methylengruppe steht,

m für 0 oder 1 steht und

n für 0 oder 1 steht,

erhält, wenn man Aminomethylheterocyclen der Formel (II),

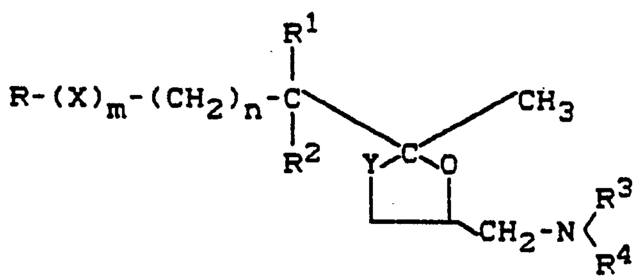

(II)

in welcher

R, $R^1$, $R^2$, $R^3$, $R^4$, X, Y, m und n die oben angegebene Bedeutung haben,

mit Saccharin gegebenenfalls in Gegenwart eines Verdünnungsmittels umsetzt.

Schließlich wurde gefunden, daß die neuen Saccharin-Salze von Aminomethylheterocyclen der allgemeinen Formel (I) eine Wirkung gegen Schädlinge besitzen.

Überraschenderweise zeigen die neuen Saccharin-Salze von Aminomethylheterocyclen der allgemeinen Formel (I) u.a. eine bessere fungizide Wirksamkeit als die aus dem Stand der Technik bekannten Saccharin-Salze von Aminoheterocyclen, wie beispielsweise das Saccharin-Salz des 5-Amino-1,2,4-triazols, welches chemisch und wirkungsmäßig naheliegende Verbindungen sind.

Die erfindungsgemäßen Saccharin-Salze von Aminomethylheterocyclen sind durch die Formel (I) allgemein definiert. Bevorzugt sind Salze der Formel (I), bei welchen

R für gegebenenfalls einfach bis fünffach, gleich oder verschieden substituiertes Phenyl oder Thienyl steht, wobei als Substituenten infrage kommen: Halogen, jeweils geradkettiges oder verzweigtes Alkyl, Alkoxy, Alkylthio, Halogenalkyl, Halogen alkoxy oder Halogenalkylthio mit jeweils 1 bis 4 Kohlenstoffatomen und gegebenenfalls 1 bis 9 gleichen oder verschiedenen Halogenatomen; oder für gegebenenfalls einfach oder mehrfach gleich oder verschieden durch niederes Alkyl substituiertes Cycloakyl mit 3 bis 7 Kohlenstoffatomen steht,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ für geradkettiges oder verzweigtes Alkyl mit 1 bis 6 Kohlenstoffatomen steht und

$R^4$ für jeweils geradkettiges oder verzweigtes Alkyl, Alkenyl oder Alkinyl mit jeweils bis zu 8 Kohlenstoffatomen steht, oder

$R^3$ und $R^4$ gemeinsam mit dem Stickstoffatom, an welches sie gebunden sind, für einen gegebenenfalls einfach oder mehrfach, gleich oder verschieden substituierten, gesättigten Heterocyclus mit 5 bis 7 Ringgliedern und 1 oder 2 Heteroatomen stehen, wobei als Heteroatome Stickstoff und Sauerstoff in Frage kommen und wobei als Sub stituenten in Frage kommen: geradkettiges oder verzweigtes Alkyl mit 1 bis 4 Kohlenstoffatomen, Hydroxymethyl und Derivate davon, wie Ether und Ester, sowie geradkettiges oder verzweigtes Alkoxycarbonyl mit 1 bis 4 Kohlenstoffatomen,

X für Sauerstoff oder Schwefel steht,

Y für Sauerstoff, Schwefel oder die Methylengruppe steht,

m für 0 oder 1 steht und

n für 0 oder 1 steht.

Besonders bevorzugt sind Salze der Formel (I), bei welchen

R für gegebenenfalls ein-bis dreifach, gleich oder verschieden substituiertes Phenyl steht, wobei als Substituenten in Frage kommen: Fluor, Chlor, Brom, Methyl, Ethyl, n-und i-Propyl, n-, i-, s-und t-Butyl, Methoxy, Ethoxy, n-und i-Propoxy, Methylthio, Trifluormethyl, Trifluormethoxy und Trifluormethylthio; oder für gegebenenfalls ein-bis fünffach, gleich oder verschieden substituiertes Cyclohexyl steht, wobei als Substituenten infrage kommen: Methyl, Ethyl, n-und i-Propyl, n-, i-, s-oder t-Butyl; oder für gegebenenfalls ein-oder zweifach, gleich oder verschieden substituiertes Thienyl steht, wobei als Substituenten infrage kommen: Fluor, Chlor, Brom, Methyl, Ethyl, n-oder i-Propyl, n-, i-, s-oder t-Butyl,

$R^1$ für Wasserstoff oder Methyl steht,

$R^2$ für Methyl oder Ethyl steht,

$R^3$ für Methyl steht und

$R^4$ für Methyl, Ethyl, n-oder i-Propyl, n-oder i-Butyl, n-oder i-Pentyl, n-oder i-Hexyl, Allyl, Butenyl, n-oder i-Pentenyl, n-oder i-Hexenyl, Propargyl, Butinyl, n-oder i-Pentinyl oder n-oder i-Hexinyl steht oder

$R^3$ und $R^4$ gemeinsam mit dem Stickstoffatom, an welches sie gebunden sind, für einen gegebenenfalls ein-bis vierfach, gleich oder verschieden substituierten Heterocyclus der Formel

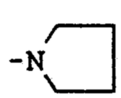 ; 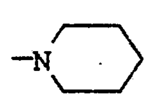 ; 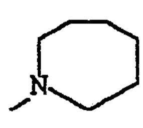 ; 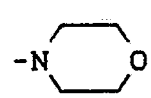 oder 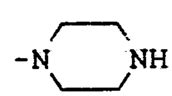

stehen, wobei als Substituenten infrage kommen: Methyl, Ethyl, Phenyl, Hydroxymethyl, Methoxymethyl, Ethoxymethyl, Acyloxymethyl, Methoxycarbonyl oder Ethoxycarbonyl,

X für Sauerstoff oder Schwefel steht,

Y für Sauerstoff, Schwefel oder die Methylengruppe steht,

m für 0 oder 1 steht und

n für 0 oder 1 steht.

Im einzelnen seien außer den bei den Herstellungsbeispielen genannten Verbindungen die folgenden Saccharin-Salze der allgemeinen Formel (I) genannt:

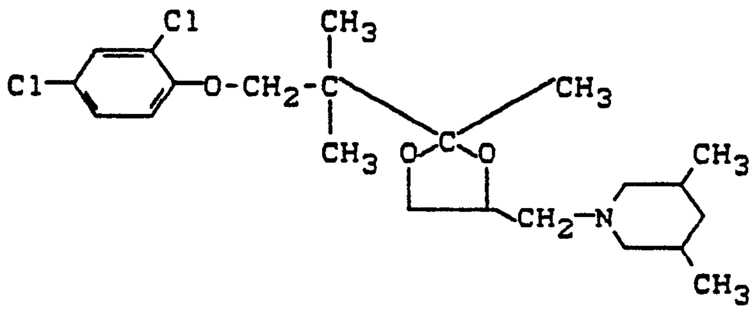 x 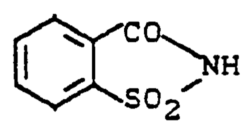

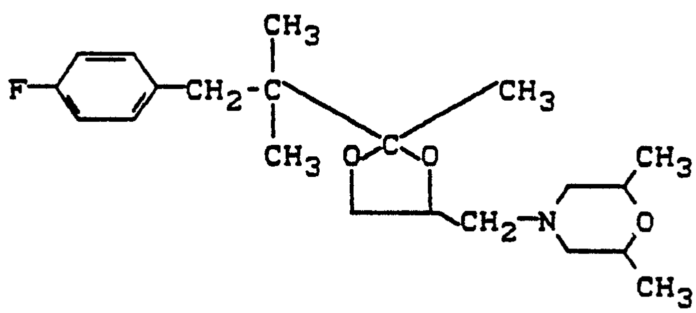 x 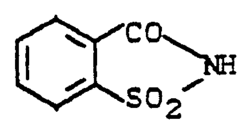

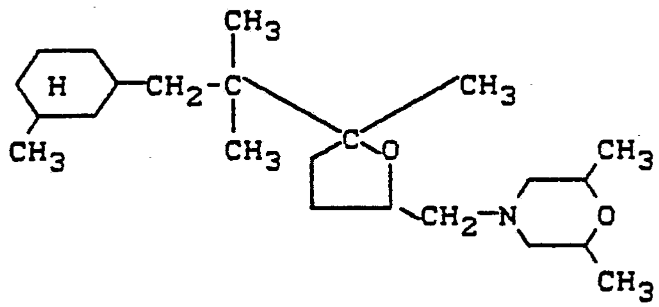 x 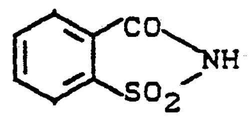

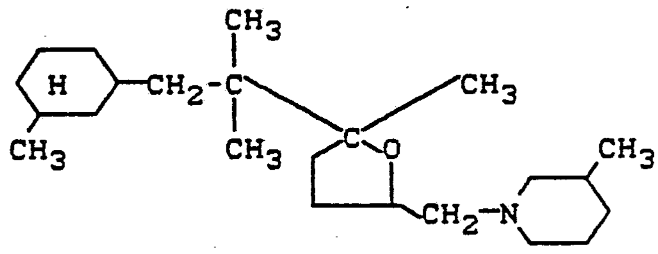 x 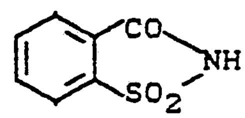

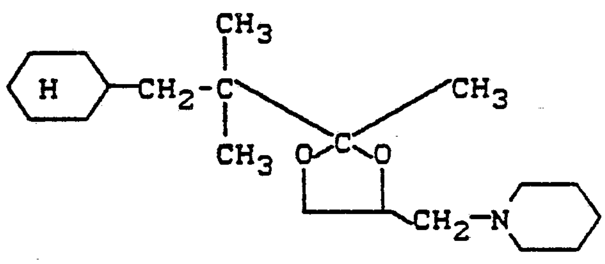 x 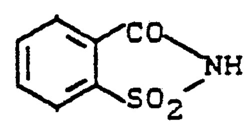

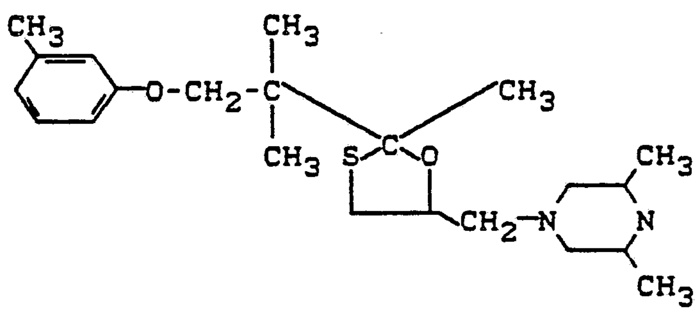 x 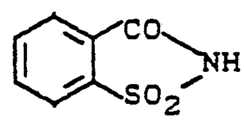

Verwendet man beispielsweise 2-[1-(3-Chlorphenoxy)-2-methyl-prop-2-yl]-2-methyl-4-[2,6-dimethyl-4-morpholinyl)-methyl]-1,3-dioxolan und Saccharin als Ausgangsstoffe, so läßt sich der Reaktionsablauf des erfindungsgemäßen Verfahrens durch das folgende Formelschema darstellen:

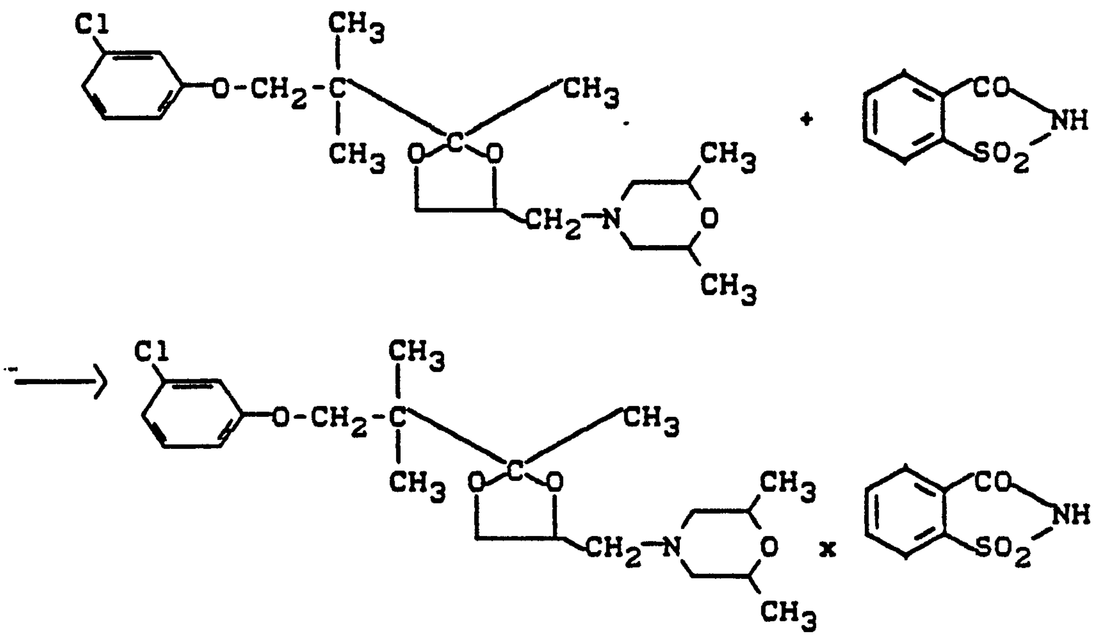

Die zur Durchführung des erfindungsgemäßen Verfahrens als Ausgangsstoffe benötigten Aminomethylheterocyclen sind durch die Formel (II) allgemein definiert. In dieser Formel (II) stehen R, $R^1$, $R^2$, $R^3$, $R^4$, X, Y, m und n für diejenigen Reste, die bereits im Zusammenhang mit der Beschreibung der erfindungsgemäßen Stoffe der Formel (I) für diese Substituenten genannt wurden.

Die Aminomethylheterocyclen der Formel (II) sind größtenteils bekannt oder lassen sich mit Hilfe bekannter Verfahren analog erhalten (vgl. z.B. EP 97 822; DE-OS 3 413 996; DE-OS 33 24 769; DE-OS 33 28 151).

Man erhält sie beispielsweise, wenn man substituierte Heterocyclen der Formel (III),

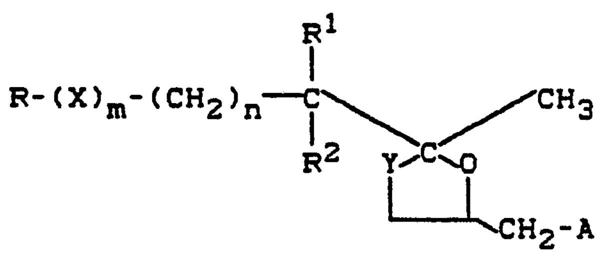

(III)

in welcher

R, $R^1$, $R^2$, X, Y, m und n die oben angegebene Bedeutung haben und

A für eine elektronenanziehende Abgangsgruppe, insbessondere für Chlor oder Brom, Methansulfonyloxy oder p-Toluolsulfonyloxy steht,

mit Aminen der Formel (IV),

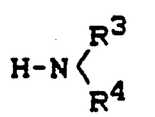

(IV)

in welcher

$R^3$ und $R^4$ die oben angegebene Bedeutung haben,

gegebenenfalls in Gegenwart eines Verdünnungsmittels, wie beispielsweise Ethanol, gegebenenfalls in

Gegenwart eines Säurebindemittels, wie beispielsweise Kaliumcarbonat, und gegebenenfalls in Gegenwart eines Katalysators, wie beispielsweise Kaliumiodid, bei Temperaturen zwischen 50 °C und 250 °C umsetzt.

Die substituierten Heterocyclen der Formel (III) sind größtenteils bekannt (vgl. z.B. EP 97 822; DE-OS 34 13 966; DE-OS 33 24 760; DE-OS 33 28 155) oder erhältlich in Analogie zu bekannten Verfahren (vgl. auch die Herstellungsbeispiele).

Die Amine der Formel (IV) sind allgemein bekannte Verbindungen der organischen Chemie.

Als Verdünnungsmittel zur Durchführung des erfindungsgemäßen Verfahrens kommen inerte organische Lösungsmittel infrage.

Hierzu gehören insbesondere Ether, wie Ethylenglykoldimethyl-oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid oder Alkohole, wie Methanol, Ethanol oder Propanol.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 100 °C, vorzugsweise bei Temperaturen zwischen 10 °C und 50 °C.

Zur Durchführung des erfindungsgemäßen Verfahrens setzt man pro Mol an Aminomethylheterocyclus der Formel (II) äquimolare Mengen an Saccharin ein. Man löst beide Reaktionspartner bei der geeigneten Reaktionstemperatur in einem geeigneten Lösungsmittel und entfernt anschließend das Lösungsmittel durch Destillation im Vakuum. Die Reinigung der so erhältlichen Salze erfolgt mit Hilfe üblicher Reinigungsmethoden, beispielsweise durch Umkristallisieren oder Umfällen. Die Charakterisierung der gelegentlich amorphen Salze erfolgt mit Hilfe spektroskopischer Verfahren (IR; NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium); Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut,

und des Bodens.

Dabei lassen sich die erfindungsgemäßen Wirkstoffe mit besonders gutem Erfolg zur Bekämpfung von Getreidekankheiten, wie beispielsweise gegen den Erreger des echten Getreidemehltaus (Erysiphe graminis), gegen den Erreger der Blattfleckenkrankheit (Pyrenophora teres), gegen Puccinia-Arten und Septoria-Arten und gegen den Erreger der Reisfleckenkrankheit (Pyriculaira oryzae) einsetzen. Hervorzuheben ist, daß die erfindungsgemäßen Wirkstoffe neben einer guten protektiven Wirksamkeit auch systemische Eigenschaften besitzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoffe-imprägnierte Natur-und synthetische Stoffe, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln, und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und orga nischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001 %.

Bei der Saatgutbehandlung werden im allgemeinen Wirkstoffmengen von 0,001 bis 50 g je Kilogramm

Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeipiele

Beispiel 1:

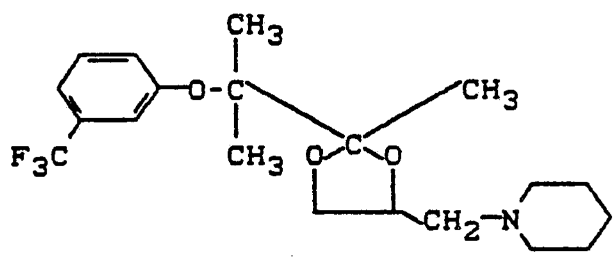

x

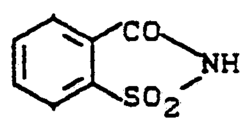

4.85 g (0,012 Mol) 2-[2-(3-Trifluormethyl-phenoxy)-prop-2-yl]-2-methyl-4-(piperidin-1-yl-methyl)-1,3-dioxolan werden zusammen mit 2,17 g (0.023 Mol) Saccharin in 50 ml Ethanol gelöst. Die klare Lösung wird im Vakuum eingedampft und der Rückstand im Hochvakuum getrocknet. Man erhält 7 g (100 % der Theorie) an 2-[2-(3-Trifluormethyl-phenoxy)-prop-2-yl]-2-methyl-4-(piperidin-1-yl-methyl)-1,3-dioxolan-Saccharin-Salz als amorphe Verbindung.

NMR ($CDCl_3$/TMS): $\delta$ = 4,4 - 4,6 (m,1H); 4,2 - 4,35 (m,1H); 3,5 - 3,7 (m,3H) ppm.

Herstellung der Ausgangsverbindungen der Formel (II)

1a)

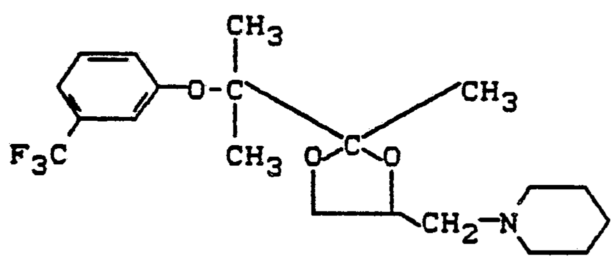

8,4 g (0,025 Mol) 2-[2-(3-Trifluormethyl-phenoxy)-prop-2-yl]-2-methyl-4-chlormethyl-1,3-dioxolan werden zusammen mit 10 g (0.11 Mol) Piperidin 14 Stunden bei 140 °C Badtemperatur gerührt. Zur Aufarbeitung nimmt man die erkaltete Reaktionsmischung in Ether auf, wäscht mehrfach mit Wasser, trocknet über Natriumsulfat, engt im Vakuum ein und destilliert den Rückstand im Hochvakuum.

Man erhält 9,3 g (96 % der Theorie) an 2-[2-(3-Trifluormethyl-phenoxy)-prop-2-yl]-2-methyl-4-(piperidin-1-yl-methyl)-1,3-dioxolan vom Brechungsindex $n_D^{20}$ 1.4711.

Herstellung der Vorprodukte der Formel (III)

1b)

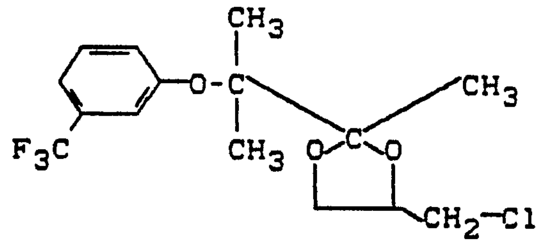

12,3 g (0,5 Mol) 3-(3-Trifluormethyl-phenoxy)-3-methylbutan-2-on werden zusammen mit 110 g (1 Mol) 3-Chlorpro pandiol und 10 g saurem Ionenaustauscher (Lewasorb A10) in 600 ml Toluol 14 Stunden über einem Wasserabscheider unter Rückfluß erhitzt. Das erkaltete Reaktionsgemisch wird filtriert, über Natriumsulfat getrocknet und eingeengt. Der Rückstand wird im Hochvakuum destilliert. Man erhält 109 g (65 % der Theorie) an 2-[2-(3-Trifluormethyl-phenoxy)-prop-2-yl]-2-methyl-4-chlormethyl-1,3-dioxolan vom Siedepunkt 163 °C bei 0,2 mbar.

Herstellung von Vorprodukten zur Herstellung von Verbindungen der Formel (III)

1c)

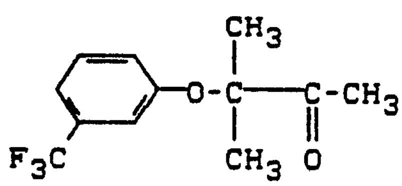

Eine Mischung aus 121 g (0,75 Mol) 3-Trifluormethylphenol, 110 g (0,75 Mol) Kaliumcarbonat und 1,5 g Tetrabutylammoniumbromid in 500 ml Butanon wird bei 60 °C unter Rühren tropfenweise mit 130 g (0,75 Mol) 2-Brom-2-methyl-butan-3-on versetzt und nach beendeter Zugabe 16 Stunden bei 80 °C gerührt. Zur Aufarbeitung wird die erkaltete Reaktionsmischung filtriert, das Filtrat im Vakuum eingeengt und im Hochvakuum destilliert.
Man erhält 137 g (74 % der Theorie) an 3-(3-Trifluormethyl-phenoxy)-3-methyl-butan-2-on vom Siedepunkt 60 °C - 62 °C bei 0,5 mbar.

Beispiel 2:

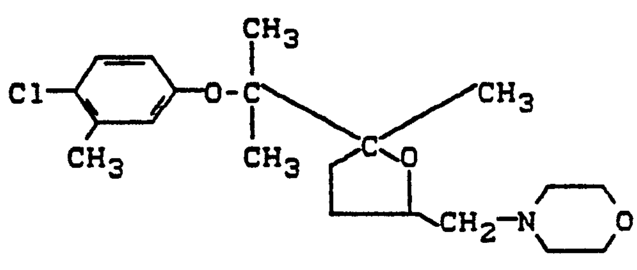

x

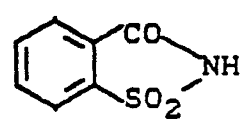

3,6 g (0,01 Mol) 2-[2-(4-Chlor-3-methylphenoxy)-prop-2-yl]-2-methyl-5-(4-morpholinylmethyl)-tetrahydrofuran werden in 50 ml Aceton mit 1,8 g (0,01 Mol) Saccharin versetzt. Die klare Lösung wird im Vakuum eingedampft und im Hochvakuum getrocknet.
Man erhält 5,4 g (100 % der Theorie) an 2-[2-(4-Chlor-3-methylphenoxy)-prop-2-yl]-2-methyl-5-(4-morpholinylmethyl)-tetrahydrofuran-Saccharin-Salz als amorphe Verbindung.
1H-NMR ($CDCl_3$/TMS): $\delta$ = 4,45 - 4,7 (m,1H); 4.07 (m,3H) ppm.

Herstellung der Ausgansverbindung der Formel (II)

2a)

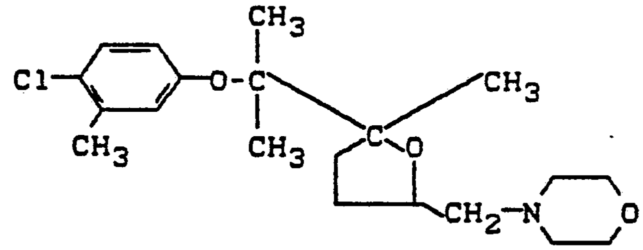

18.5 g (0,05 Mol) 2-Brommethyl-5-[2-(4-chlor-3-methylphenoxy)-prop-2-yl]-5-methyl-tetrahydrofuran werden zusammen mit 14 g (0,16 Mol) Morpholin 13 Stunden bei einer Badtemperatur von 130 °C gerührt. Zur Aufarbeitung nimmt man die erkaltete Reaktionsmischung in Ether auf, wäscht mehrfach mit Wasser, trocknet über Natriumsulfat, engt im Vakuum ein und reinigt den Rückstand chromatographisch (Kieselgel; Laufmittel: Petrolether/Essigester 1:1).
Man erhält 12,7 g (68 % der Theorie) an 2-[2-(4-Chlor-3-methylphenoxy)-prop-2-yl]-2-methyl-5-(4-morpholinylmethyl)-tetrahydrofuran mit dem Brechungsindex $n_D^{20}$ 1.5139.

Herstellung des Vorproduktes der Formel (III)

2b)

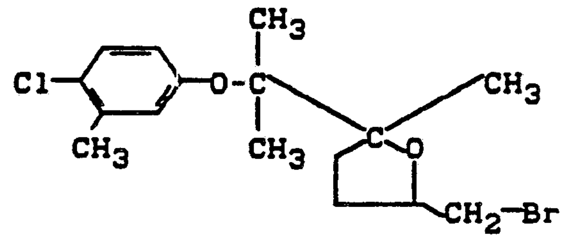

88,8 g (0,3 Mol) 6-(4-Chlor-3-methylphenoxy)-5-hydroxy-5,6,6-trimethyl-1-hexen in 600 ml absolutem Chloroform werden portionsweise unter Rühren und Kühlung mit 60 g (0,37 Mol) N-Bromsuccinimid versetzt, so daß die Reaktionstemperatur 40 °C nicht übersteigt. Nach beendeter Zugabe rührt man weitere 14 Stunden bei Raumtemperatur, wäscht dann zweimal mit Wasser, trocknet über Natriumsulfat und entfernt das Lösungsmittel im Vakuum. Man erhält 94 g (82% der Theorie) an 2-Brommethyl-5-[2-(4-chlor-3-methylphenoxy)-prop-2-yl]-5-methyl-tetrahydrofuran, das ohne Reinigung weiter umgesetzt wird.

Herstellung des Vorproduktes zu Verbindungen der Formel (III)

2c)

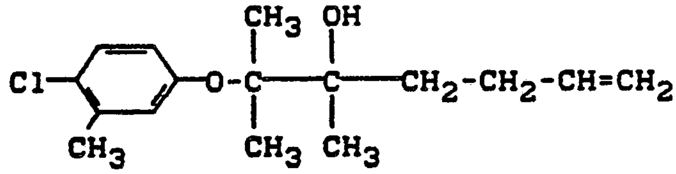

Zu einer Lösung aus 10 g (0,4 Mol) Magnesium und 48 g (0,4 Mol) Allylbromid in 400 ml absolutem Ether tropft man unter Rühren und Kühlung 101 g (0,42 Mol) 2-[2-(4-Chlor-3-methyl-phenoxy)-prop-2-yl]-2-methyl-oxiran. Nach beendeter Zugabe erwärmt man für 4 Stunden auf Siedetemperatur und gibt anschließend vorsichtig wässerige Ammoniumchloridlösung zur erkalteten Reaktionsmischung, bis keine Gasentwicklung mehr auftritt. Die organische Phase wird abgetrennt, über Natriumsulfat getrockent, im Vakuum eingeengt und im Hochvakuum destilliert.
Man erhält 97 g (86 % der Theorie) an 6-(4-Chlor-3-methyl-phenoxy)-5-hydroxy-5,6,6-trimethyl-1-hexen vom

Siedepunkt 118 °C - 120 °C bei 0,01 mbar.

Herstellung des Vorproduktes von 2c

2d)

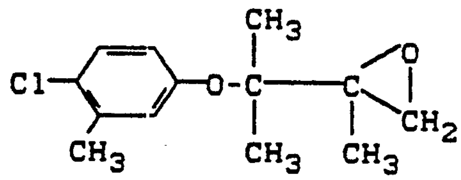

Zu einer Suspension von 100 g (0,45 Mol) Trimethylsulfoxoniumiodid in 100 g Dimethylsulfoxid gibt man innerhalb von 10 Minuten 23 g (0,42 Mol) Natriummethylat, verdünnt dann mit 200 ml absolutem Tetrahydrofuran, rührt 3 Stunden bei Raumtemperatur, versetzt anschließend mit 104 g (0,46 Mol) 2-(4-Chlor-3-methyl-phenoxy)-2-methyl-butan-3-on und rührt 32 Stunden bei Raumtemperatur. Der ausgefallene Feststoff wird abfiltriert, das Filtrat im Vakuum eingeengt, der Rückstand in Dichlormethan aufgenommen, mit Wasser gewaschen, über Natriumsulfat getrocknet und im Vakumm vom Lösungsmittel befreit.
Man erhält 86,7g (85 % der Theorie) an 2-[2-(4-Chlor-3-methyl-phenoxy)-prop-2-yl]-2-methyl-oxiran als Öl, welches ohne Reinigung weiter umgesetzt wird.

Herstellung des Vorproduktes von 2d

2e)

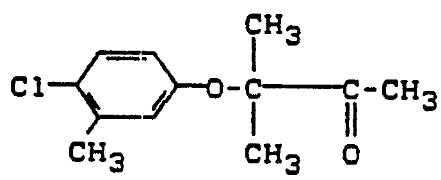

Eine Mischung aus 107 g (0,75 Mol) 4-Chlor-3-methylphenol, 110 g Kaliumcarbonat und 1,5 g Tetraubtuylammoniumbromid in 500 ml Butanon wird bei 60 °C unter Rühren tropfenweise mit 130 g (0,75 Mol) 2-Brom-2-methyl-butan-3-on versetzt und nach beendeter Zugabe 16 Stunden bei 80 °C gerührt. Zur Aufarbeitung wird die erkaltete Reaktionsmischung filtriert, das Filtrat im Vakuum eingeengt und im Hochvakuum destilliert.
Man erhält 110 g (65 % der Theorie) an 3-(4-Chlor-3-methyl-phenoxy)-3-methyl-butan-2-on vom Siedepunkt 108°C / 0,5 mbar.

Beispiel 3:

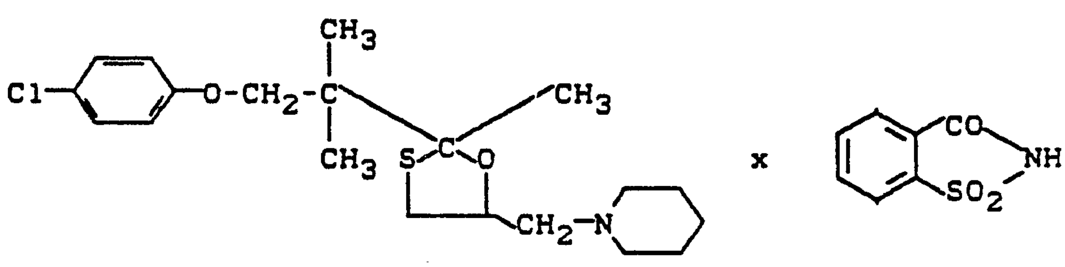
x

96 g (0,025 Mol) 2-[1-(4-Chlorphenoxy)-2-methyl-prop-2-yl]-2-methyl-5-(piperidin-1-yl-methyl)-1,3-oxathiolan werden zusammen mit 4,6 g (0,025 Mol) Saccharin in 40 ml Tetrahydrofuran gelöst. Man entfernt das Lösungsmittel im Vakuum und trocknet, den Rückstand im Hochvakuum. Man erhält 14,1 g (98 % der Theorie) an 2-[1-(4-Chlorphenoxy)-2-methyl-prop-2-yl]-2-methyl-5-(piperidin-1-yl-methyl)-1,3-oxathiolan-Saccharin-Salz als amorphe Substanz.
$^1$H-NMR ($CDCl_3$/TMS: $\delta$ = 4,2 - 4,4 (m,1H); 3,6 - 3,95 (m,3H) ppm.

Herstellung der Ausgangsverbindung der Formel (II)

3a)

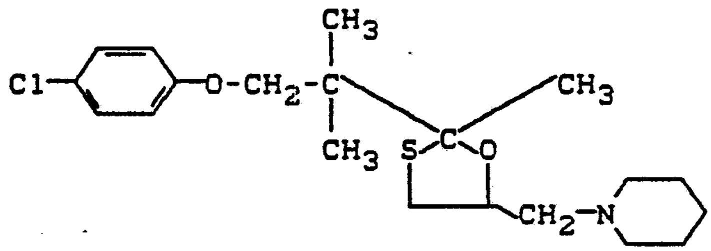

10 g (0,03 Mol) 5-Chlormethyl-2-(4-chlorphenoxy)-2-methyl-prop-2-yl]-2-methyl-1,3-oxathiolan und 10 g (0,12 Mol) Piperidin werden 12 Stunden lang auf 120 °C erhitzt. Nach dem Erkalten wird das Reaktionsgemisch mit Essigester verdünnt, zweimal mit Wasser gewaschen, über Natriumsulfat getrocknet und im Vakuum eingeengt. Der Rückstand wird einer Kugelrohrdestillation unterzogen (Kp: 200 °C/0,13 mbar) oder über eine Kieselgelsäule mit einem Laufmittelgemisch aus Petrolether/Essigester 2:1 chromatographiert.
Man erhält 8 g (69,5 % der Theorie) an 2-(1-(4-Chlorphenoxy)-2-methyl-prop-2-yl]-2-methyl-5-piperidin-1-yl-methyl-1,3-oxathiolan vom Brechungsindex $n_D^{20}$ = 1,5440.

Herstellung des Vorproduktes der Formel (III) zu 3a

3b)

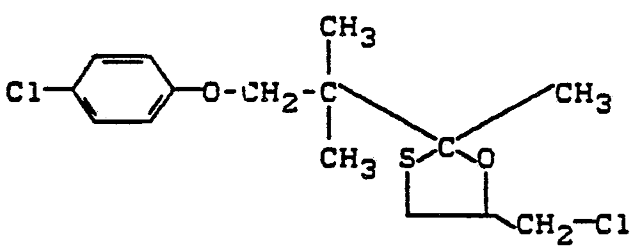

Zu einer siedenden Lösung von 80,7 g (0,35 Mol) 4-(4-Chlorphenoxy)-3,3-dimethyl-butan-2-on (vgl. z. B. DE-OS 30 21 516) und 45 g (0,35 Mol) 1-Chlor-3-mercapto-2-propanol in 240 ml absolutem Ether tropft man 50 g (0,35 Mol) Bortrifluorid-Etherat. Nach beendeter Zugabe kocht man weitere 90 Minuten am Rückfluß. Die abgekühlte Reaktionsmischung wird 2 mal mit je 100 ml 0,1 molarer Natriumhydrogencarbonat-und einmal mit gesättigter Natriumchloridlösung gewaschen, über Natriumsulfat getrocknet und im Vakuum vom Lösungsmittel befreit. Der Rückstand wird im Vakuum destilliert.
Man erhält 50 g (43 % der Theorie) an 5-Chlormethyl-2-[1-(4-chlorphenoxy)-2-methyl-prop-2-yl]-2-methyl-1,3-oxathiolan vom Siedepunkt 160 °C bei 0,3 mbar.

Beispiel 4:

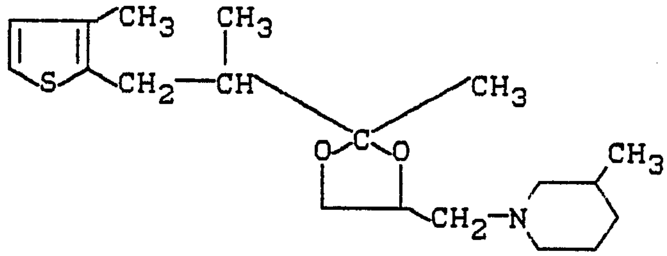

x

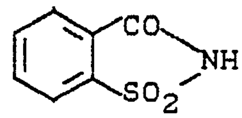

3,3 g (0,01 Mol) 2-Methyl-4-(3-methylpiperidin-1-yl-methyl)-2-[1-(3-methylthien-2-yl)-propan-2-yl]-dioxolan und 1,8 g (0,01 Mol) Saccharin werden zusammen in 50 ml Aceton gelöst und 10 Minuten bei Raumtemperatur gerührt. Das Lösungsmittel wird im Vakuum abdestilliert, der Rückstand mit Ether verrieben und nach Abdekantieren getrocknet.
Man erhält 4,9 g (96 % der Theorie) an 2-Methyl-4-(3-methylpiperidin-1-yl-methyl)-2-[1-(3-methylthien-2-yl)-propan-2-yl]-dioxolan-Saccharin-Salz vom Schmelzpunkt 39 °C - 41 °C.

Herstellung der Ausgangsverbindung der Formel (II) zu 4

4a)

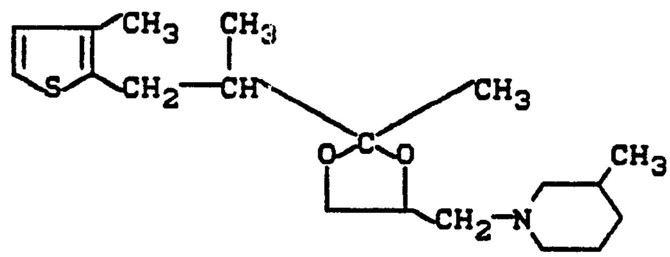

15 g (0,05 Mol) 2-Methyl-4-(3-methylpiperidin-1-yl-methyl)-2-[1-(3-methylthien-2-yl]-propen-2-yl]-dioxolan in 150 ml Methanol werden mit 10 g Palladium auf Kohle (5prozentig) versetzt und 6 Stunden bei 80 °C und einem Druck von 120 bar hydriert. Zur Aufarbeitung filtriert man den Katalysator ab und entfernt das Lösungsmittel im Vakuum.
Man erhält 14,5 g (96 % der Theorie) an 2-Methyl-4-(3-methylpiperidin-1-yl-methyl)-2-[1-(3-methylthien-2-yl)-propan-2-yl]-dioxolan mit dem Brechungsindex $n_D^{20}$ = 1.5139.

Herstellung des Vorproduktes zur Herstellung von 4a

4b)

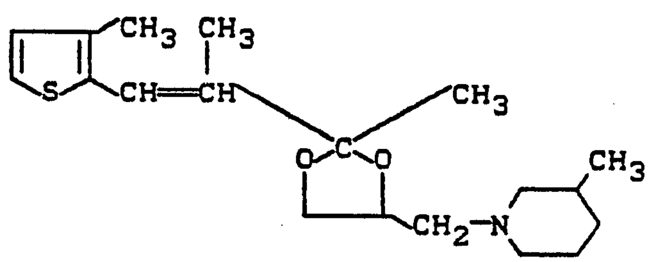

Eine Mischung aus 19 g (0,07 Mol) 4-Chlormethyl-2-methyl-2-[1-(3-methylthien-2-yl)-propen-2-yl]-dioxolan und 18 g (0,18 Mol) 3-Methylpiperidin wird unter Rühren 16 Stunden auf 120 °C erhitzt. Zur Aufarbeitung wird die erkaltete Reaktionsmischung zwischen Wasser und Diethylether verteilt, die organische Phase abgetrennt, über Magnesiumsulfat getrocknet und im Vakuum eingedampft. Man erhält 19 g (81 % der Theorie) an 2-Methyl-4-(3-methylpiperidin-1-yl-methyl)-2-[1-(3-methylthien-2-yl]-propen-2-yl]-dioxolan

vom Brechungsindex $n_D^{20}$ 1.5402.

Herstellung der Ausgangsverbindung zur Herstellung von 4b

4c)

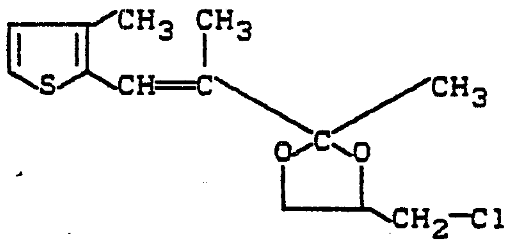

78 g (0,43 Mol) 2-Methyl-1-(3-methylthien-2-yl)-buten-3-on und 100 g (0,9 Mol) 3-Chlor-propan-1,2-diol in 600 ml Toluol werden unter Zusatz von 4 g saurem Ionenaus tauscher (Lewasorb A10) 16 Stunden unter Rückfluß über einem Wasserabscheider erhitzt. Zur Aufarbeitung filtriert man die erkaltete Reaktionsmischung, wäscht das Filtrat mit Wasser, trocknet über Natriumsulfat und entfernt das Lösungsmittel im Vakuum.
Man erhält 77 g (65 % der Theorie) an 4-Chlormethyl-2-methyl-2-[1-(3-methylthien-2-yl)-propen-2-yl]-dioxolan vom Brechungsindex $n_D^{20}$ 1.5430.

Herstellung der Ausgangsverbindung zur Herstellung von 4c

4d)

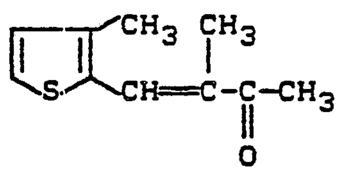

Zu einer Mischung aus 100 g (0,79 Mol) 3-Methyl-thiophen-2-aldehyd und 300 ml absolutem Methylethylketon gibt man 100 ml einer Lösung von Chlorwasserstoff in Ether (ca. 9prozentig) und rührt 24 Stunden bei Raumtemperatur. Zur Aufarbeitung entfernt man die flüchtigen Bestandteile im Vakuum und kristalliert den Rückstand aus Ether um.
Man erhält 82 g (57 % der Theorie) an 2-Methyl-1-(3-methylthien-2-yl)-buten-3-on vom Schmelzpunkt 72 °C.

In entsprechender Weise und gemäß den allgemeinen Angaben zur Herstellung erhält man die folgenden Saccharin-Salze von Aminomethylheterocyclen der allgemeinen Formel (I):

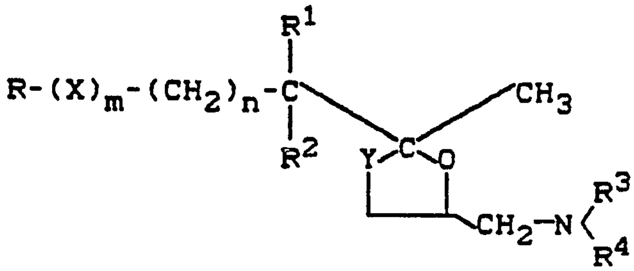

x

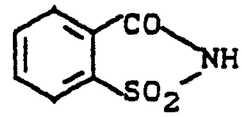 (I)

| Bsp. Nr. | 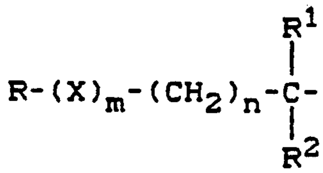 | Y | 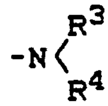 | 1H-NMR*) |
|---|---|---|---|---|
| 5 | 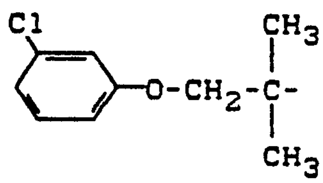 | O | 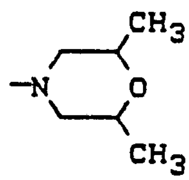 | |
| 6 | 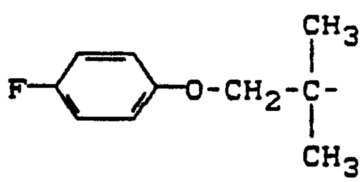 | O | 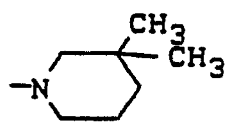 | |
| 7 | 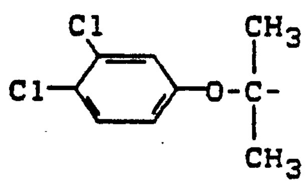 | O | 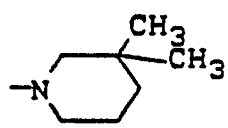 | |
| 8 | 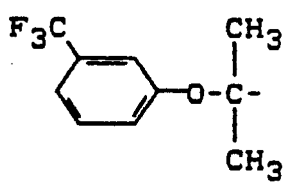 | O | 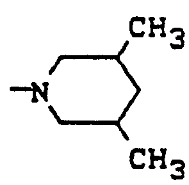 | |
| 9 | 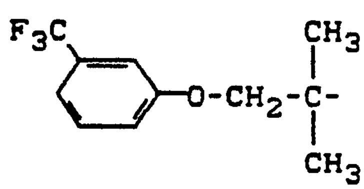 | O | 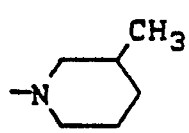 | 4,5;4,9(m,1H) |

| Bsp. Nr. | $R-(X)_m-(CH_2)_n-C(R^1)(R^2)-$ | Y | $-N(R^3)(R^4)$ | 1H-NMR*) |
|---|---|---|---|---|
| 10 | 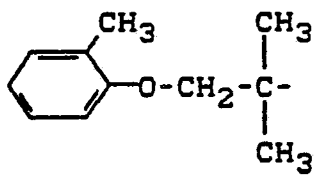 | O | 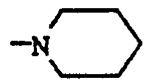 | |
| 11 | 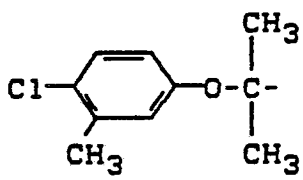 | $CH_2$ | 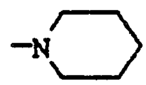 | |
| 12 | 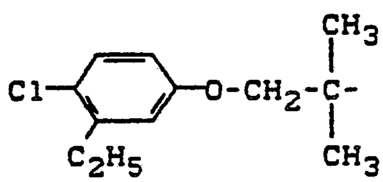 | O | 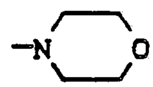 | 4,55;4,81(m,1H); 4,15 (m,4H) |
| 13 | 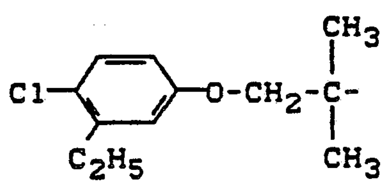 | O | 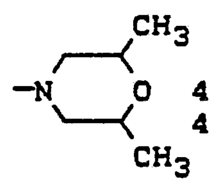 | 4,55;4,81(m,1H); 4,1-4,4(m,3H) |
| 14 | 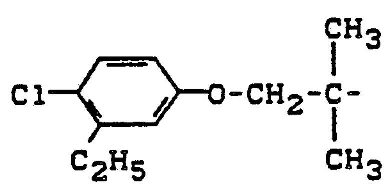 | O | 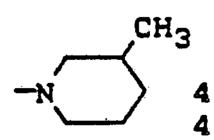 | 4,55;4,85(m,1H); 4,2-4,4(m,1H); 3,4-4,0(m,6H) |
| 15 | 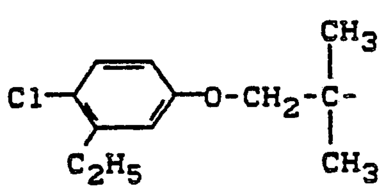 | O | 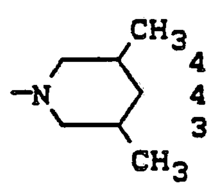 | 4,5;4,8(m,1H); 4,2-4,4(m,1H); 3,4-3,8(m,6H) |

| Bsp. Nr. | $R-(X)_m-(CH_2)_n-C(R^1)(R^2)-$ | Y | $-N(R^3)(R^4)$ | 1H-NMR*) |
|---|---|---|---|---|
| 16 | 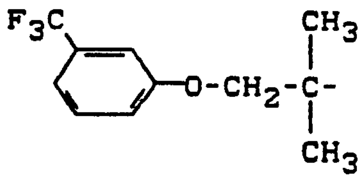 | O | 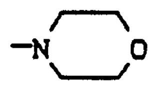 | 4,55;4,85(m,1H); 4,2-4,4(m,1H); 4,1(m,4H) |
| 17 | 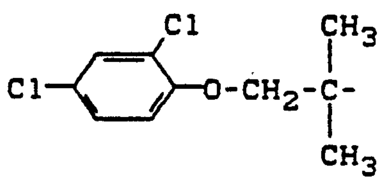 | $CH_2$ | 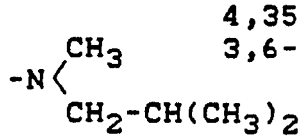 | 4,35-4,55(m,1H); 3,6-3,95(m,6H) |
| 18 | 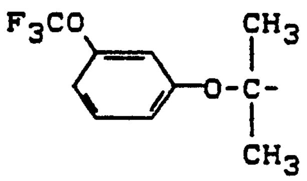 | O | 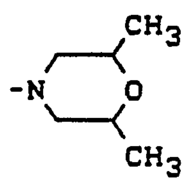 | 4,6-4,9(m,1H); 4,1-4,5(m,4H); 3,4-3,85(m,5H) |
| 19 | 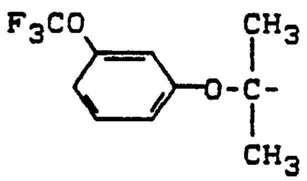 | O | 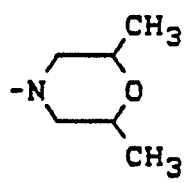 | 4,6-4,9(m,1H); 4,2-4,5(m,1H); 3,4-3,9(m,4H) |
| 20 | 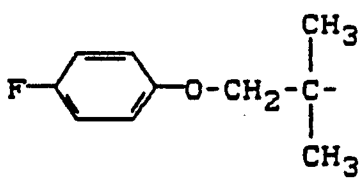 | S | 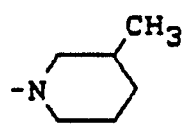 | 4,7-4,9-(m,1H); 3,5-4,0(m,4H) |
| 21 | 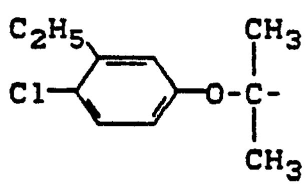 | O | 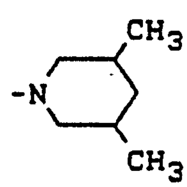 | |

| Bsp. Nr. | $R-(X)_m-(CH_2)_n-C(R^1)(R^2)-$ | Y | $-N(R^3)(R^4)$ | 1H-NMR*) |
|---|---|---|---|---|
| 22 | 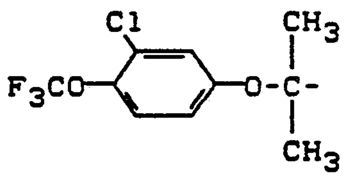 | O | 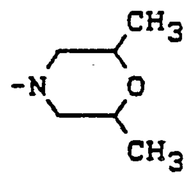 | 4,7-5,0(m,1H); 4,1-4,5(m,4H) |
| 23 | 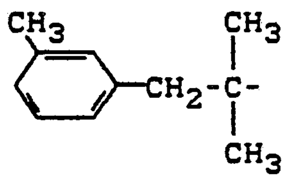 | $CH_2$ | 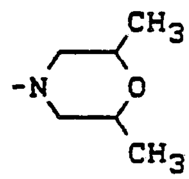 | Fp. 37 °C- 42 °C |
| 24 | 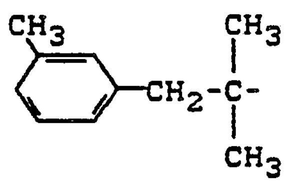 | $CH_2$ | 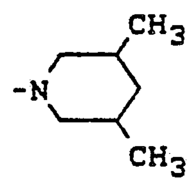 | Fp. 36 °C |
| 25 | 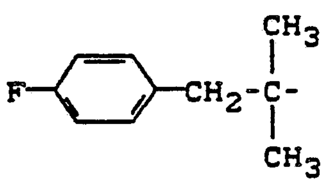 | O | 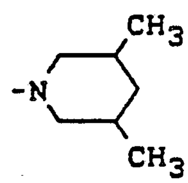 | 4,0-5,1(m,2H); 3,25-4,0(m,4H) |
| 26 | 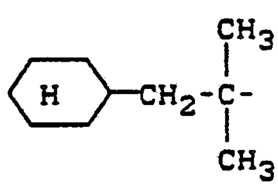 | O | 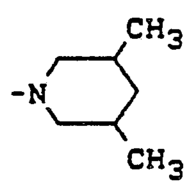 | 4,0,4,9(m,2H); 3,2-3,8(m,4H) |
| 27 | 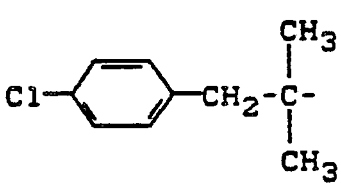 | O | 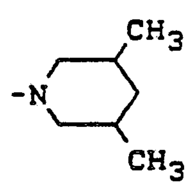 | 4,1-5,0(m,2H); 3,2-3,8(m,4H) |

| Bsp. Nr. | 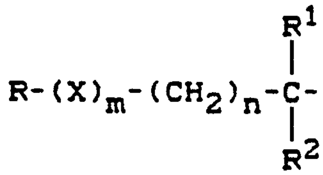 | Y | 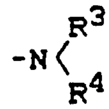 | 1H-NMR*) |
|---|---|---|---|---|
| 28 | 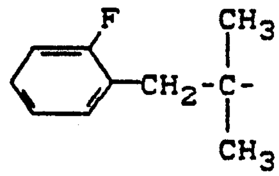 | O | 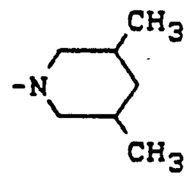 | 4,1-5,0(m,2H); 3,4-4,0(m,4H) |
| 29 | 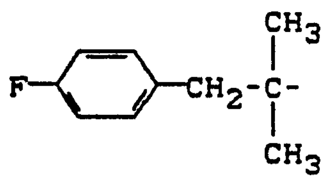 | O | 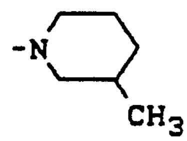 | 4,1-4,95(m,2H); 3,4-4,0(m,4H) |
| 30 | 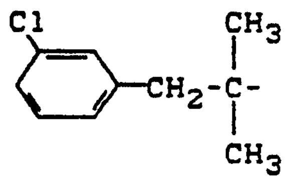 | O | 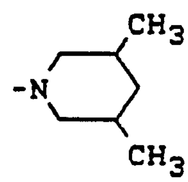 | 4,1-4,95(m,2H); 3,2-3,9(m,4H) |
| 31 | 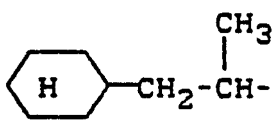 | O | 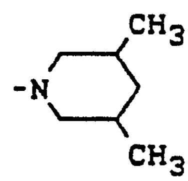 | 4,0-4,8(m,4H) 2,6-3,8( ,8H) |
| 32 | 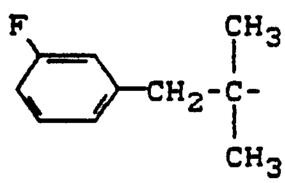 | O | 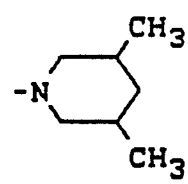 | 4,1-5,0(m,2H); 3,4-4,0(m,4H) |
| 33 | 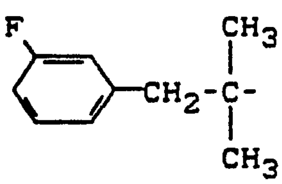 | O | 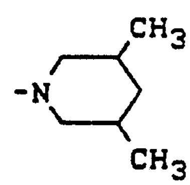 | 3,9-5,0(m,3H); 3,2-3,9(m,3H) |

| Bsp. Nr. | $R-(X)_m-(CH_2)_n-C(R^1)(R^2)-$ | Y | $-N(R^3)(R^4)$ | 1H-NMR*) |
|---|---|---|---|---|
| 34 | 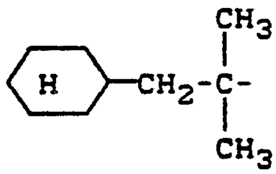 | O | 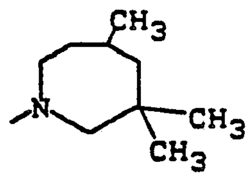 | 4,0-4,9(m,2H); 2,5-3,8(m,7H) |
| 35 | 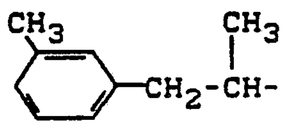 | O | 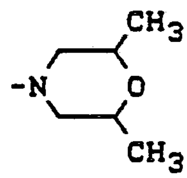 | 4,0-4,9(m,2H); 3,4-3,9(m,4H) |
| 36 | 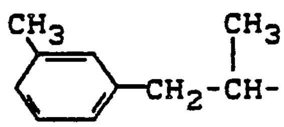 | O | 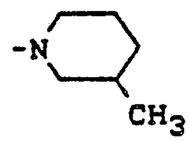 | 4,2-4,9(M,2H); 3,4-4,0(m,4H) |
| 37 | 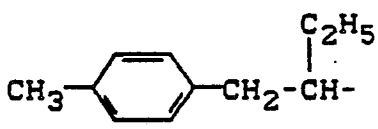 | O | 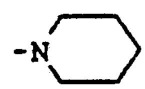 | 4,1-4,9(m,2H); 3,4-3,7(m,4H) |
| 38 | 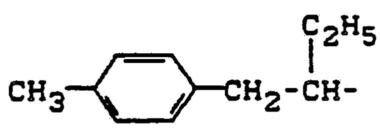 | O | 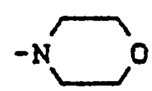 | 3,8-4,9(m,4H); 3,1-3,7(m,4H) |
| 39 | 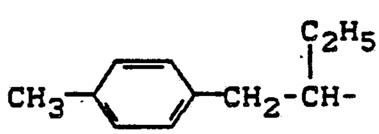 | O | 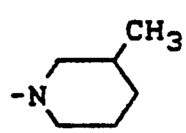 | 4,1-4,8(m,2H); 3,3-3,7(m,4H) |
| 40 | 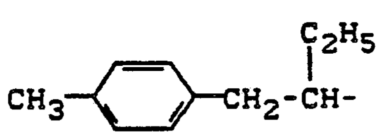 | O | 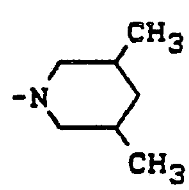 | 4,1-4,8(m,2H); 3,4-3,8(m,4H) |

| Bsp. Nr. | 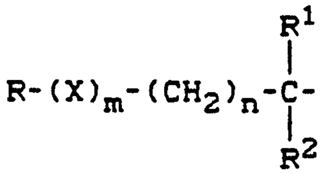 | Y | 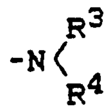 | 1H-NMR*) |
|---|---|---|---|---|
| 41 | 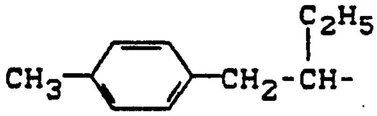 | O | 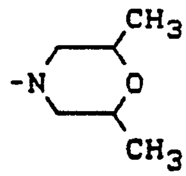 | 3,9-4,8(m,3H); 3,4-3,7(m,4H) |
| 42 | 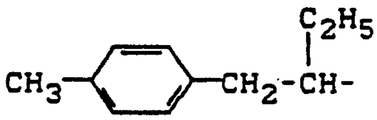 | O | 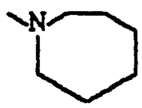 | 4,1-4,8(m,2H); 3,0-3,7(m,4H) |
| 43 | 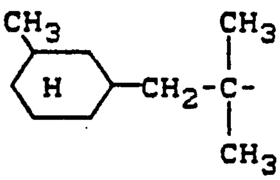 | O | 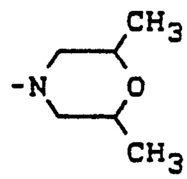 | 4,45;4,8(m,1H); 4,2-4,35(m,3H); 3,4-3,8(m,3H) |
| 44 | 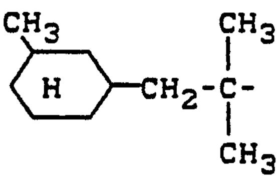 | O | 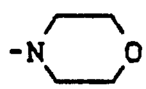 | 4,5;4,8(m,1H); 4,05-4,3(m,5H) |
| 45 | 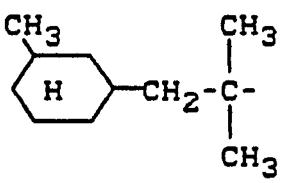 | O | 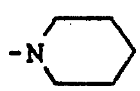 | 4,5;4,8(m,1H); 4,2-4,3(m,1H); 3,4-3,75(m,4H) |
| 46 | 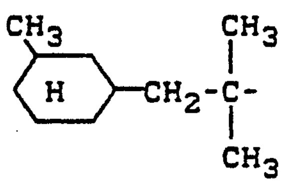 | O | 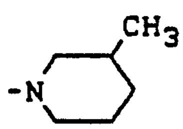 | 4,8;4,5(m,H); 4,2-4,4(m,1H); 3,4-3,8(m,4H) |

| Bsp. Nr. | $R-(X)_m-(CH_2)_n-C(R^1)(R^2)-$ | Y | $-N(R^3)(R^4)$ | 1H-NMR*) |
|---|---|---|---|---|
| 47 | 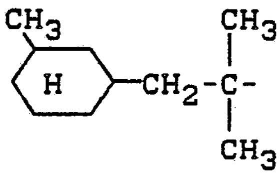 | O | 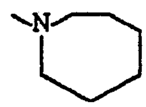 | 4,8;4,5(m,1H); 4,2-4,35(m,1H); 3,4-3,8(m,4H) |
| 48 | 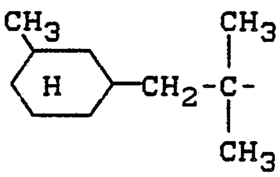 | O | 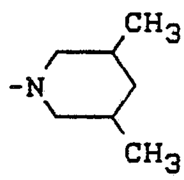 | 4,8;4,5(m,1H); 4,2-4,35(m,1H); 3,4-38(m,4H) |
| 49 | 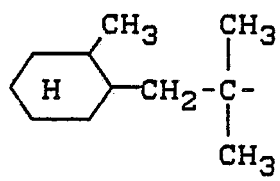 | O | 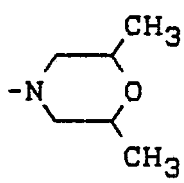 | 4,8;4,5(m,1H); 4,1-4,4(m,4H); 3,4-3,8(m,4H) |
| 50 | 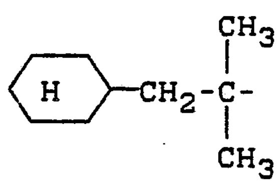 | O | 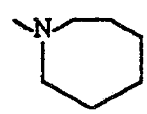 | 4,8;4,5(m,1H); 4,2-4,4(m,1H); 3,4-3,9(m,4H) |
| 51 | 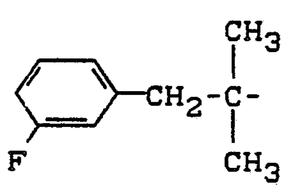 | O | 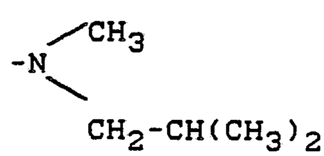 | |

| | | | |
|---|---|---|---|
| 52 | 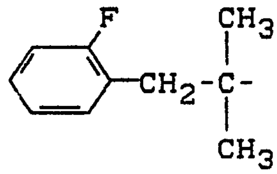 | O | 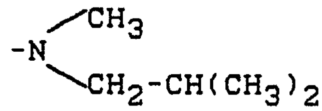 |
| 53 | 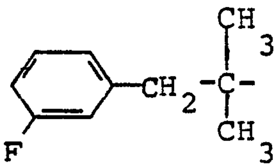 | O | 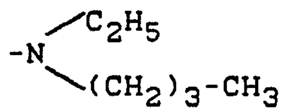 |
| 54 | 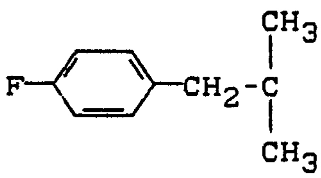 | O | 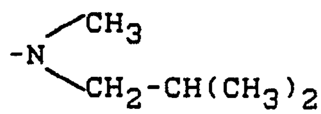 |

*) Die 1H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichssubstanzen eingesetzt:

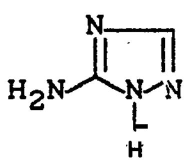 x 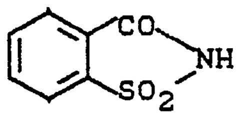 (A)

5-Amino-1,2,4-triazol-Saccharin-Salz (bekannt aus EP 158 074) und

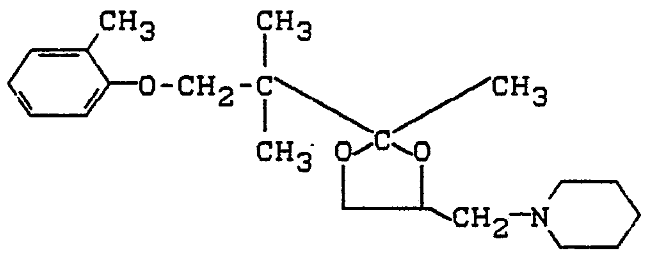

(B)

2-[1-(2-Methylphenoxy)-2-methyl-prop-2-yl]-2-methyl-4-(piperidin-1-yl-methyl)-1,3-dioxolan (bekannt aus EP 97 822).

Beispiel A

Erysiphe-Test (Gerste) / protektiv Lösungsmittel: 100 Gewichtsteile Dimethylformamid
Emulgator: 0,25 = Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen: 1, 5, 6, 25, 26, 27, 28, 29, 30, 31, 32, 34.

Beispiel B

Pyricularia-Test (Reis) /protektiv Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß den Herstellungsbeispielen: 2, 7, 8, 13, 14, 15.

Beispiel C

Pyricularia-Test (Reis) /systemisch Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegeben Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß den Herstellungsbeispielen: 2, 7, 8, 10, 13, 14, 15, 25, 26, 27, 28, 33, 35.

**Ansprüche**

1. Saccharin-Salze von Aminomethylheterocyclen der allgemeinen Formel (I),

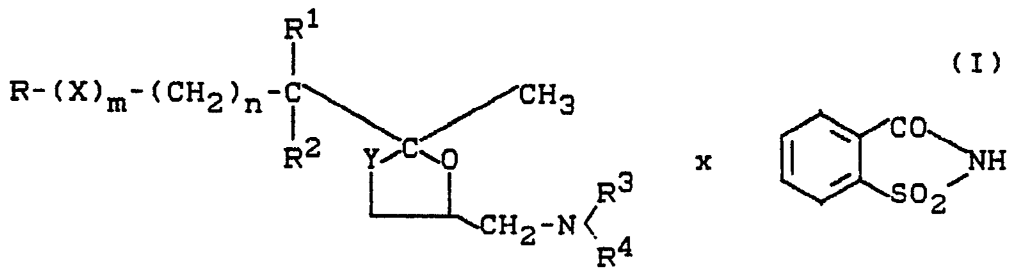

(I)

in welcher

R für gegebenenfalls substituiertes Cycloalkyl, für gegebenenfalls substituiertes Aryl oder für gegebenenfalls substituiertes Thienyl steht,

$R^1$ für Wasserstoff oder Alkyl steht,

$R^2$ für Alkyl steht,

$R^3$ für Alkyl steht und

$R^4$ für Alkyl, Alkenyl oder Alkinyl steht, oder

$R^3$ und $R^4$ gemeinsam mit dem Stickstoffatom, an welches sie gebunden sind, für einen gegebenenfalls substituierten, gesättigten Heterocyclus stehen, der weitere Heteroatom enthalten kann,

X für Sauerstoff oder Schwefel steht,

Y für Sauerstoff, Schwefel oder die Methylengruppe steht,

m für 0 oder 1 steht und

n für 0 oder 1 steht.

2. Saccharin-Salze von Aminomethylheterocyclen der allgemeinen Formel (I) gemäß Anspruch 1, in welcher

R für gegebenenfalls einfach bis fünffach, gleich oder verschieden substituiertes Phenyl oder Thienyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen; oder für gegebenenfalls einfach oder mehrfach, gleich oder verschieden durch niederes Alkyl substituiertes Cycloalkyl mit 3 bis 7 Kohlenstoffatomen steht,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ für geradkettiges oder verzweigtes Alkyl mit 1 bis 6 Kohlenstoffatomen steht und

$R^4$ für jeweils geradkettiges oder verzweigtes Alkyl, Alkenyl oder Alkinyl mit jeweils bis zu 8 Kohlenstoffatomen steht,

X für Sauerstoff oder Schwefel steht,

Y für Sauerstoff, Schwefel oder die Methylengruppe steht,

m für 0 oder 1 steht und

n für 0 oder 1 steht.

3. Saccharin-Salze von Aminomethylheterocyclen der allgmeinen Formel (I) gemäß Anspruch 1, in welcher

R für gegebenenfalls einfach bis fünffach, gleich oder verschieden substituiertes Phenyl oder Thienyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen; oder für gegebenenfalls einfach oder mehrfach, gleich oder verschieden durch niederes Alkyl substituiertes Cycloalkyl mit 3 bis 7 Kohlenstoffatomen steht,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ und $R^4$ gemeinsam mit dem Stickstoffatom, an welches sie gebunden sind, für einen gegebenenfalls einfach oder mehrfach, gleich oder verschieden substituierten, gesättigten Heterocyclus mit 5 bis 7 Ringgliedern und 1 oder 2 Heteroatomen stehen, und wobei als Substituenten in Frage kommen: geradkettiges oder verzweigtes Alkyl mit 1 bis 4 Kohlenstoffatomen, Hydroxymethyl und Derivate davon, sowie geradkettiges oder verzweigtes Alkoxycarbonyl mit 1 bis 4 Kohlenstoffatomen,

X für Sauerstoff oder Schwefel steht,

Y für Sauerstoff, Schwefel oder die Methylengruppe steht,

m für 0 oder 1 steht und

n für 0 oder 1 steht.

4. Saccharin-Salze von Aminomethylheterocyclen der allgemeinen Formel (I) gemäß Anspruch 1, in welcher

R für gegebenenfalls ein-bis dreifach, gleich oder verschieden substituiertes Phenyl steht, wobei als Substituenten in Frage kommen: Fluor, Chlor, Brom, Methyl, Ethyl, n-und i-Propyl, n-, i-, s-und t-Butyl, Methoxy, Ethoxy, n-und i-Propoxy, Methylthio, Trifluormethyl, Trifluormethoxy und Trifluormethylthio; oder für gegebenenfalls ein bis fünffach, gleich oder verschieden substituiertes Cyclohexyl steht, wobei als Substituenten infrage kommen: Methyl, Ethyl, n-und i-Propyl, n-, i-, s-oder t-Butyl; oder für gegebenenfalls

ein-oder zweifach, gleich oder verschieden substituiertes Thienyl steht, wobei als Substitutenten infrage kommen: Fluor, Chlor, Brom, Methyl, Ethyl, n-oder i-Propyl, n-, i-, s-oder t-Butyl,

$R^1$ für Wasserstoff oder Methyl steht,

$R^2$ für Methyl oder Ethyl steht,

$R^3$ für Methyl steht und

$R^4$ für Methyl, Ethyl, n-oder i-Propyl, n-oder i-Butyl, n-oder i-Pentyl, n-oder i-Hexyl, Allyl, Butenyl, n-oder i-Pentenyl, n-oder i-Hexenyl, Propargyl, Butinyl, n-oder i-Pentinyl oder n-oder i-Hexinyl steht,

X für Sauerstoff oder Schwefel steht,

Y für Sauerstoff, Schwefel oder die Methylengruppe steht,

m für 0 oder 1 steht und

n für 0 oder 1 steht.

5. Saccharin-Salze von Aminomethylheterocyclen der allgemeinen Formel (I) gemäß Anspruch 1, in welcher

R für gegebenenfalls ein-bis dreifach, gleich oder verschieden substituiertes Phenyl steht, wobei als Substituenten in Frage kommen: Fluor, Chlor, Brom, Methyl, Ethy, n-und i-Propyl, n-, i-, s-und t-Butyl, Methoxy, Ethoxy, n-und i-Propoxy, Methylthio, Trifluormethyl, Trifluormethoxy und Trifluormethylthio; oder für gegebenenfalls ein bis fünffach, gleich oder verschieden substituiertes Cyclohexyl steht, wobei als Substituenten infrage kommen: Methyl, Ethyl, n-und i-Propyl, n-, i-, s-oder t-Butyl; oder für gegebenenfalls ein-oder zweifach, gleich oder verschieden substituiertes Thienyl steht, wobei als Substituenten infrage kommen: Fluor, Chlor, Brom, Methyl, Ethyl, n-oder i-Propyl n-, i-, s-oder t-Butyl,

$R^1$ für Wasserstoff oder Methyl steht,

$R^2$ für Methyl oder Ethyl steht,

$R^3$ und $R^4$ gemeinsam mit dem Stickstoffatom, an welches sie gebunden sind, für einen gegebenenfalls ein-bis vierfach, gleich oder verschieden substituierten Heterocyclus der Formel

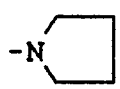 ; 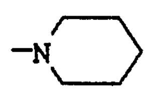 ; 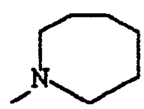 ; 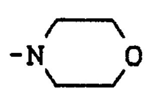 oder 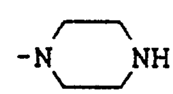

stehen, wobei als Substituenten infrage kommen: Methyl, Ethyl, Phenyl, Hydroxymethyl, Methoxymethyl, Ethoxymethyl, Acyloxymethyl, Methoxycarbonyl oder Ethoxycarbonyl,

X für Sauerstoff oder Schwefel steht,

Y für Sauerstoff, Schwefel oder die Methylengruppe steht,

m für 0 oder 1 steht und

n für 0 oder 1 steht.

6. Verfahren zur Herstellung von Saccharin-Salzen von Aminomethylheterocyclen der allgemeinen Formel (I),

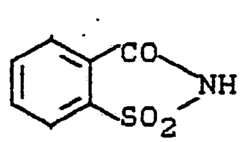

x

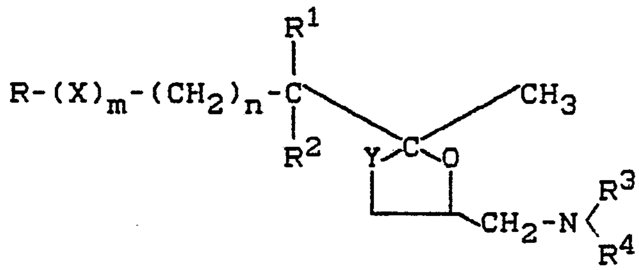

(I)

in welcher

R für gegebenenfalls substituiertes Cycloalkyl, für gegebenenfalls substituiertes Aryl oder für gegebenenfalls substituiertes Thienyl steht,

$R^1$ für Wasserstoff oder Alkyl steht,

$R^2$ für Alkyl steht,

$R^3$ für Alkyl steht und

$R^4$ für Alkyl, Alkenyl oder Alkinyl steht oder

$R^3$ und $R^4$ gemeinsam mit dem Stickstoffatom, an welches sie gebunden sind, für einen gegebenenfalls substituierten, gesättigten Heterocyclus stehen, der weitere Heteroatome enthalten kann,

X für Sauerstoff oder Schwefel steht,

Y für Sauerstoff, Schwefel oder die Methylengruppe steht,

m für 0 oder 1 steht und

n für 0 oder 1 steht,

dadurch gekennzeichnet, daß man Aminomethylheterocyclen der Formel (II),

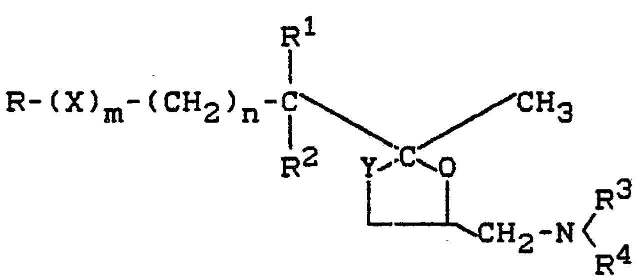

(II)

in welcher

R, $R^1$, $R^2$, $R^3$, $R^4$, X, Y, m und n die oben angegebene Bedeutung haben,

mit Saccharin gegebenenfalls in Gegenwart eines Verdünnungsmittels umsetzt.

7. Schädlingsbekämpfungsmittel, gekennzeichnet durch einen Gehalt an mindestens einem Saccharin-Salz von Aminomethylheterocyclen der Formel (I) gemäß den Ansprüchen 1 und 6.

8. Verwendung von Saccharin-Salzen von Aminomethylheterocyclen der Formel (I) gemäß den Ansprüchen 1 und 6 zur Bekämpfung von Schädlingen.

9. Verfahren zur Bekämpfung von Schädlingen, dadurch gekennzeichnet, daß man Saccharin-Salze von Aminomethylheterocyclen der Formel (I) gemäß den Ansprüchen 1 und 6 auf Schädlinge oder ihren Lebensraum einwirken läßt.

10. Verfahren zur Herstellung von Schädlingsbekämpfungsmitteln, dadurch gekennzeichnet, daß man Saccharin-Salze von Aminomethylheterocyclen der Formel (I) gemäß den Ansprüchen 1 und 6 mit Streckmitteln und/oder oberflächenaktiven Mitteln vermischt.

Europäisches Patentamt

# EUROPÄISCHER RECHERCHENBERICHT

Nummer der Anmeldung

EP 87 11 6564

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 158 074 (BAYER AG)<br>* Ansprüche; Seite 8, Zeile 18 - Seite 9, Absatz 1 * | 1,6-10 | C 07 D 275/06<br>C 07 D 317/28<br>C 07 D 307/14<br>C 07 D 327/04<br>C 07 D 405/06<br>C 07 D 411/06<br>C 07 D 413/06<br>A 01 N 43/80<br>A 01 N 43/40<br>A 01 N 43/28<br>A 01 N 43/84<br>A 01 N 43/08 |
| A | EP-A-0 131 793 (BAYER AG)<br>* Ansprüche 1, 5-9; Zusammenfassung * & DE - A - 3 324 769 (Kat. D,A) | 1,7-10 | |
| A | EP-A-0 133 950 (BAYER AG)<br>* Ansprüche 1, 6-9; Zusammenfassung * & DE - A - 3 328 151 (Kat. D,A) | 1,7-10 | |
| D,A | DE-A-3 413 996 (BAYER AG)<br>* Ansprüche 1, 5-10; Zusammenfassung * | 1,7-10 | |
| D,A | EP-A-0 097 822 (BAYER AG)<br>* Ansprüche 1, 10 * | 1,7 | |
| A | FR-A-2 163 322 (DELALANDE S.A.)<br>* Anspruch 1 * | 1 | **RECHERCHIERTE SACHGEBIETE (Int. Cl.4)**<br>C 07 D 275/00 |

Der vorliegende Recherchenbericht wurde für alle Patentansprüche erstellt

| Recherchenort | Abschlußdatum der Recherche | Prüfer |
|---|---|---|
| BERLIN | 08-02-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument